# FASCICULE DE BREVET EUROPEEN

(11) **EP 2 593 438 B1**
(45) Date de publication et mention de la délivrance du brevet: **06.09.2017**
(21) Numéro de dépôt: 11743846.5
(22) Date de dépôt: 12.07.2011
(51) Int. Cl.: C07D 233/36, C07D 403/12, C08F 8/30, C08J 3/24

(54) **MOLECULES PORTEUSES DE GROUPES ASSOCIATIFS**
MOLEKÜLE MIT KOMBINIERBAREN GRUPPEN
MOLECULES HAVING COMBINABLE GROUPS

(30) Priorité: 13.07.2010 FR 1055718
(43) Date de publication de la demande: 22.05.2013
(73) Titulaire: Arkema France, 92700 Colombes (FR)
(72) Inventeur: SEEBOTH, Nicolas, F-63000 Clermont-Ferrand (FR); IVANOV, Serguey, Orekhovo-Zouevo 142611 (RU); COUTURIER, Jean-Luc, F-69006 Lyon (FR); HIDALGO, Manuel, F-69530 Brignais (FR)
(86) Numéro de dépôt international: PCT/FR2011/051652
(87) Numéro de publication internationale: WO 2012/007685

(56) Documents cités:
- WO-A1-2010/031956
- US-A1- 2004 010 090

## Description

La présente invention concerne des molécules associatives azotées comprenant au moins un motif les rendant susceptibles de s'associer entre elles ou avec une charge par des liaisons non covalentes, et comprenant une fonction capable de réagir avec un polymère contenant des insaturations pour former une liaison covalente avec ledit polymère.

Les polymères modifiés comportant des groupements associatifs le long de la chaîne polymérique sont des polymères comprenant au moins un motif les rendant susceptibles de s'associer entre elles ou avec une charge par des liaisons non covalentes. Un avantage de ces polymères est que ces liaisons physiques sont réversibles sous l'influence de facteurs extérieurs tels que la température ou le temps de sollicitation par exemple. Ainsi, les propriétés mécaniques de ces polymères modifiés sont modulables en fonction des paramètres de l'environnement d'utilisation.

De tels polymères sont par exemple décrits dans le document publié sous le numéro WO2010/031956.

Ce document décrit des élastomères comprenant des chaînes polymères souples associées entre elles d'une part par des ponts de réticulation permanente à liaisons covalentes et d'autre part par des ponts de réticulation à liaisons non covalentes. Les molécules greffées sur les élastomères comportent des groupements associatifs à base d'hétérocycle azoté permettant l'établissement des liaisons physiques. Parmi les groupements associatifs envisagés dans ce document sont cités les groupes imidazolidinyle, triazolyle, triazinyle, bis-uréyle, uréido-pyrimidyle.

Pour modifier les élastomères, on peut le faire réagir avec une molécule comportant d'une part le groupement associatif et d'autre part un groupement réactif formant une liaison covalente avec une fonction réactive portée par l'élastomère. Ceci implique donc une fonctionnalisation préalable de l'élastomère.

Il s'avère en outre, que les élastomères ainsi modifiés comportent une certaine proportion de fonctions n'ayant pas réagi et qui influe sur les propriétés finales du matériau.

C'est la raison pour laquelle des recherches ont été menées sur d'autres procédés de modification de polymères pour introduire des groupements associatifs le long de la chaîne.

Le but de la présente invention est donc de proposer une alternative de modification de polymères applicables également à des polymères ne comportant pas de fonctions réactives.

Ce but est atteint en ce que les inventeurs viennent de découvrir que de nouvelles molécules comprenant à la fois au moins un groupement associatif et au moins un groupement réactif, permettant de modifier un polymère, comprenant au moins une double liaison, sans qu'il ne soit nécessaire que le polymère en question comporte des fonctions réactives.

L'invention concerne un composé comprenant au moins un groupement Q, et au moins un groupement A reliés entre eux par au moins et de préférence un groupement « espaceur » Sp dans lequel :
- Q comprend un motif azo-dicarbonyle,
- A comprend un groupe associatif comprenant au moins un atome d'azote,
- Sp est un atome ou un groupe d'atomes formant une liaison entre Q et A.

Selon l'invention, le groupe fonctionnel azo-dicarbonyle, répond à la formule :

W-CO-N=N-CO-

dans laquelle W représente
un groupement de formule :

R'-Z-,

dans lequel :
• -Z représente un atome d'oxygène, de soufre ou un groupe -NH ou -NR',
• -R' représente un groupe alkyle en C₁-C₂₀,
ou
un groupement de formule :

-Sp'-A'

dans laquelle :
- Sp', identique ou différent de Sp est un groupement espaceur divalent reliant le groupe fonctionel azodicarbonylé à un autre groupe associatif A', et Sp' est une chaîne alkyle linéaire ou ramifiée en C₁-C₂₄, éventuellement comprenant un ou plusieurs hétéroatomes choisis parmi les atomes d'azote de soufre, de silicium.
- A' , identique ou différent de A, est un groupe associatif comprenant au moins un atome d'azote.

Selon l'invention, le groupe associatif comprenant au moins un atome d'azote, répond à la formule (II) suivante : 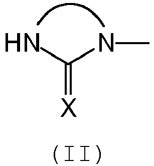 où X désigne un atome d'oxygène ou de soufre.

Selon l'invention, le groupement espaceur Sp est une chaîne alkyle linéaire ou ramifiée en C₁-C₂₄, éventuellement comprenant un ou plusieurs hétéroatomes choisis parmi les atomes d'azote de soufre, de silicium ou d'oxygène.

Un polymère greffé par un composé tel que défini ci-dessus est mélangé à des charges ; celui-ci n'établit que des liaisons labiles avec les charges, ce qui permet d'assurer une bonne interaction polymère - charge, bénéfique pour les propriétés finales du polymère, mais sans les inconvénients qu'une trop forte intéraction polymère - charge pourrait provoquer.

Par « groupe associatif », on entend des groupes susceptibles de s'associer les uns aux autres par des liaisons hydrogène, ioniques et/ou hydrophobes. Il s'agit selon un mode préféré de l'invention de groupes susceptibles de s'associer par des liaisons hydrogène.

Lorsque les groupes associatifs sont susceptibles de s'associer par des liaisons hydrogène, chaque groupe associatif comporte au moins un « site » donneur et un site accepteur vis-à-vis de la liaison hydrogène de sorte que deux groupes associatifs identiques sont auto-complémentaires et peuvent s'associer entre eux en formant au moins deux liaisons hydrogène.

Les composés selon l'invention comportant un groupement Q, un groupement « espaceur » et un groupement associatif peuvent par exemple être représentés par la formule (Ia) suivante :

A-Sp-Q (Ia).

Les composés selon l'invention comportant un groupement Q, un groupement « espaceur » et deux groupements associatifs peuvent par exemple être représentés par la formule (Ib) suivante :

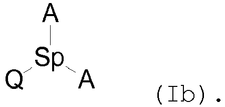

De manière similaire, les composés selon l'invention comportant deux groupements Q, un groupement « espaceur » et un groupement associatif peuvent par exemple être représentés par la formule (Ic) suivante :

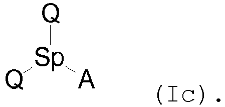

Selon le même principe, les composés selon l'invention comportant deux groupements Q, un groupement « espaceur » et deux groupements associatifs peuvent par exemple être représentés par la formule (Id) suivante :

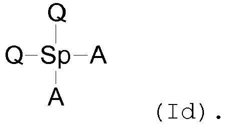

Selon l'invention, le groupement A répond à la formule (II) suivante : 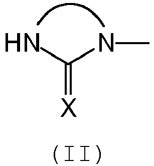 où X désigne un atome d'oxygène ou de soufre, de préférence un atome d'oxygène.

De préférence, le groupement A comprend un hétérocycle di ou triazoté, généralement à 5 ou 6 atomes, de préférence di azoté, et comprenant au moins une fonction carbonyle, de manière encore plus préférée, le groupement A comprend un groupe imidazolidinyle de formule (II).

Les composés objets de l'invention sont représentés par les formules (VIII) ou (IX) :

R'-Z-CO-N=N-CO-Sp-A (VIII)

ou

A'-Sp'-CO-N=N-CO-Sp-A (IX)

dans lesquelles :
R', Z, Sp, A, Sp' et A' sont tels que définis précédemment et A, Sp et Sp' peuvent comporter un ou plusieurs hétéroatomes.

Le groupement « espaceur » Sp permet de relier au moins un groupement Q et/ou au moins un groupement associatif, et ainsi peut être de tout type connu en soi. Le groupement « espaceur » ne doit cependant pas, ou peu, interférer avec les groupements Q et associatif du composé selon l'invention.

Ledit groupement « espaceur » est donc considéré comme un groupement inerte vis-à-vis du groupement Q qui de préférence ne présente pas de fonctions alcényle susceptibles de réagir avec ce groupement.

Le groupement « espaceur » est de préférence une chaîne hydrocarbonée, linéaire, ramifiée, cyclique, peut contenir un ou plusieurs radicaux aromatiques, et/ou un ou plusieurs hétéroatomes. Ladite chaîne peut éventuellement être substituée, pour autant que les substituants soient inertes vis-à-vis des groupements Q.

Selon un mode de réalisation préféré, le groupement « espaceur » est une chaîne alkyle linéaire ou ramifiée en C1-C24, de préférence C1-C10, plus préférentiellement une chaîne alkyle linéaire en C1-C6, éventuellement comprenant un ou plusieurs hétéroatomes choisis parmi les atomes d'azote, de soufre, de silicium ou d'oxygène.

De manière tout à fait préférée, le groupement « espaceur » Sp ou Sp' est choisi parmi -(CH2)y-, -NH-(CH2)y-, -O-(CH2)y-, y étant un entier de 1 à 6.

De préférence, le composé objet de l'invention est choisi parmi les composés de formule (X) ou (XI) suivantes : 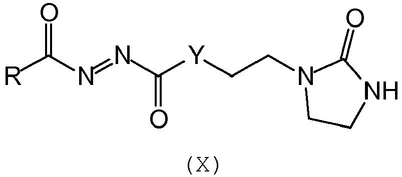 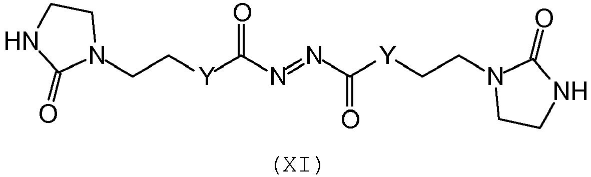 dans lesquelles :
- Y représente un groupe divalent choisi parmi un groupe méthylène, un atome d'oxygène, de soufre et un groupe - NH-, et
- R représente un groupe alcoxyle en C1-C6, de préférence un alcoxyle en C1-C4 et de manière tout à fait préférée le méthoxyle ou l'éthoxyle.

De manière encore plus préférentielle le composé selon l'invention peut être choisi parmi les composés de formules (XII) à (XV) ci-dessous :

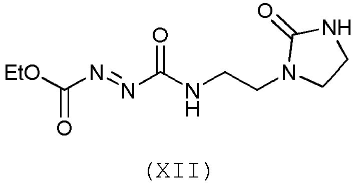

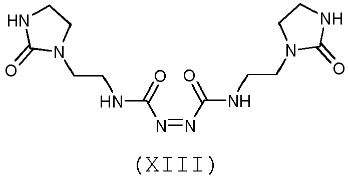

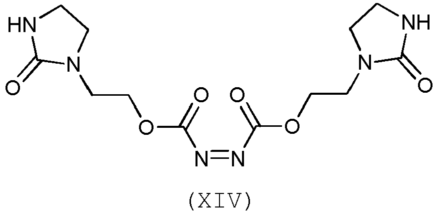

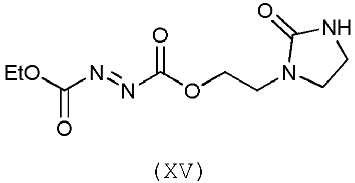

Les composés objets de l'invention peuvent être préparés en trois étapes selon le schéma général suivant : 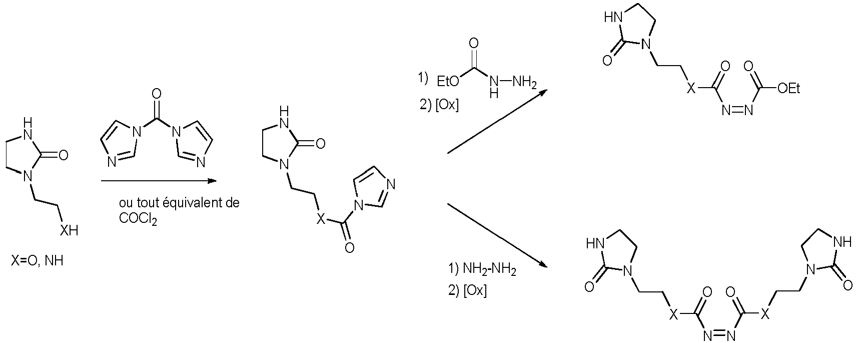 ou encore par réaction directe sur un dialkylazodicarboxylate ou un dialkylhydrazodicarboxylate selon le schéma réactionnel suivant :

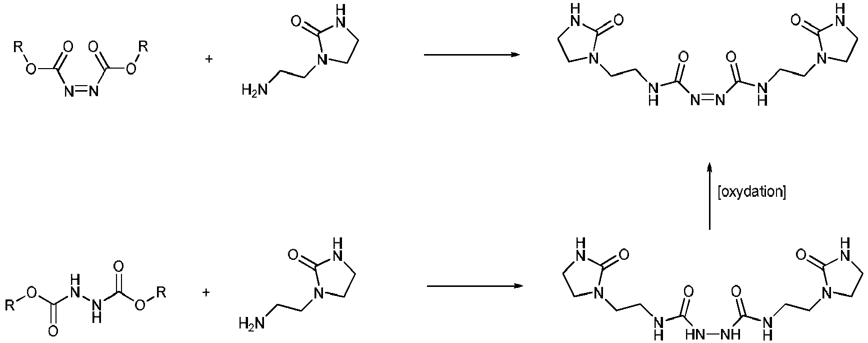

Les exemples suivants sont présentés à titre d'illustration, d'autres voies de synthèse ou des améliorations de celles décrites ci-dessous étant envisageables.

### Exemple 1 : Préparation du (E)-ethyl 2-(2-(2-oxoimidazolidin-1-yl)ethylcarbamoyl)diazenecarboxylate

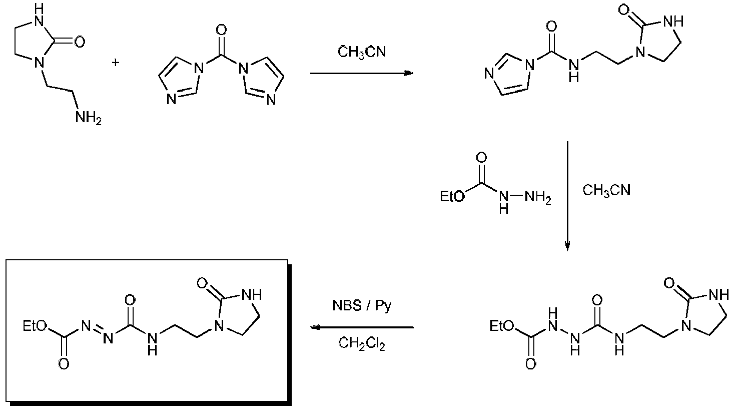

### a) Préparation du N-(2-(2-oxoimidazolidin-1-yl)ethyl)-1H-imidazole-1-carboxamide

La N-(2-(2-oxoimidazolidin-1-yl)ethyl)-1H-imidazole-1-carboxamide est préparée selon le mode opératoire suivant :

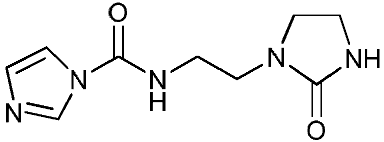

A une solution de 1-(2-aminoethyl)imidazolidin-2-one (46.5g, 0.36 mol) dans l'acétonitrile anhydre (750mL) est ajouté en une fois le carbonyldiimidazole (64.2g, 0.4 mol). Le milieu réactionnel est ensuite agité pendant 3 à 5 heures à température ambiante. Le précipité obtenu est filtré et lavé sur le filtre par de l'acétonitrile sec (3 fois 40 mL) et l'éther de pétrole (2 fois 50 mL, coupe 40/60°C) et enfin séché pendant 10 - 15 heures à température ambiante.

Un solide blanc (74.5 g, rendement 93 %) de point de fusion 154 °C est obtenu.

La pureté molaire est de 88 % mol. (RMN ¹H). Caractérisation RMN ¹H, ¹³C, ¹⁵N

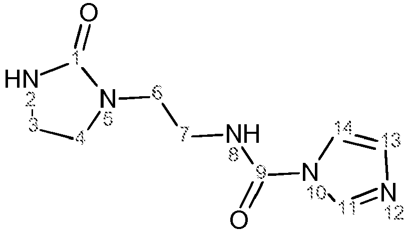

| **Atome** | **δ ¹H (ppm) + mult.** | **δ ¹³C (ppm)** | **δ ¹⁵N (ppm)** |
|---|---|---|---|
| **1** | - | 162.4 | - |
| **2** | 6.26 (s) | - | -302.7 (¹J_{1H-15N} = 90Hz) |
| **3** | 3.15 (t) | 37.5 | - |
| **4** | 3.34 (t) | 44.7 | - |
| **5** | - | - | -299.2 |
| **6** | 3.17 (t) | 42.5 | - |
| **7** | 3.28 (t) | 38.4 | - |
| **8** | 8.53 | - | -286.3 (¹J_{1H-15N} = 90Hz) |
| **9** | - | 148.8 | - |
| **10** | - | - | -185.1 |
| **11** | 8.14 (s) | 135.9 | - |
| **12** | - | - | -112.6 |
| **13** | 6.95 (s) | 139.5 | - |
| **14** | 7.57 (s) | 116.6 | - |

Solvant utilisé : DMSO - Calibration sur le signal du DMSO à 2.44 ppm en ¹H, 39.5 ppm en ¹³C et sr=19238,46 en ¹⁵N

### b) Préparation de l'ethyl 2-(2-(2-oxoimidazolidin-1-yl)ethylcarbamoyl)hydrazinecarboxylate

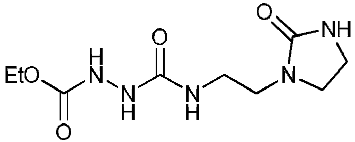

Au N-(2-(2-oxoimidazolidin-1-yl)ethyl)-1H-imidazole-1-carboxamide (74,0 g, 0,33 mol, pureté 88 % mol par RMN) dans l'acétonitrile anhydre (750 mL) est ajouté en une fois l'ethyl hydrazinecarboxylate (38,0 g, 0,36 mol). Le milieu réactionnel est agité pendant 3 heures à 70-75 °C puis pendant 2-3 heures à température ambiante.

Le précipité est filtré et lavé par de l'acétonitrile (2 fois 50 mL) et de l'éther de pétrole (2 fois 50 mL, coupe 40/60°C) et enfin séché pendant 10 - 15 heures à température ambiante.

Un solide blanc (79,6 g, rendement 93 %) de point de fusion 179 °C est obtenu.

La pureté molaire est supérieure à 99 % (RMN 1H).

Caractérisation RMN 1H, 13C, 15N

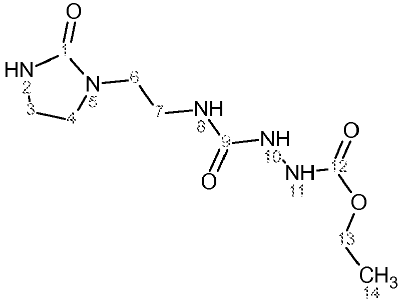

| **Atome** | **δ ¹H (ppm) + mult.** | **δ ¹³C (ppm)** | **δ ¹⁵N (ppm)** |
|---|---|---|---|
| **1** | - | 162.4 | - |
| **2** | 6.20 | - | -303.1 (¹J_{1H-15N} = 90Hz) |
| **3** | 3.13 (t) | 37.6 | - |
| **4** | 3.28 (t) | 45.0 | - |
| **5** | - | - | -298.2 |
| **6** | 2.99 (t) | 43.4 | - |
| **7** | 3.04 (t) | 37.9 | - |
| **8** | 6.33/7.69/8.30/8.68* | - | -301.3* |
| **9** | - | 158.3 | - |
| **10** | 6.33/7.69/8.30/8.68* | - | -301.3* |
| **11** | 6.33/7.69/8.30/8.68* | - | -301.3* |
| **12** | - | 156.9 | - |
| **13** | 3.96 (q) | 60.4 | - |
| **14** | 1.11 (t) | 14.6 | - |

| | | | |
|---|---|---|---|
| * Les protons 8, 10 et 11 étant des groupements NH, leur déplacement chimique ¹H ne peut être exactement attribué. Le déplacement chimique ¹³C correspond au groupement 8. | | | |

Solvant utilisé : DMSO - Calibration sur le signal du DMSO à 2.44 ppm en ¹H, 39.5 ppm en ¹³C et sr=19238,46 en ¹⁵N

### c) Préparation d'ethyl 2-(2-(2-oxoimidazolidin-1-yl)ethylcarbamoyl)diazenecarboxylate, composé selon l'invention

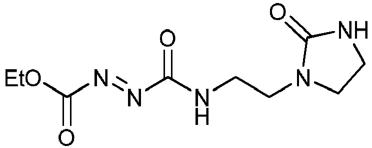

A un mélange de pyridine (3,05 g, 0,039 mol) et d'hydrazinecarboxylate (10,00 g, 0,039 mol) dans le dichlorométhane (200 mL), refroidi à 5 - 10 °C est ajouté en une fois le N-bromosuccinimide (6,87 g, 0,039 mol) dans le dichlorométhane (100 mL). Le milieu réactionnel est agité pendant 1 heure à 10 °C puis la phase organique est lavée par de l'eau (2 fois 150 mL). La phase organique est ensuite séchée pendant 15 minutes sur Na₂SO₄ puis les solvants sont évaporés sous pression réduite (T_{bain} 18 °C, 40-50 mbar). De l'éther diéthylique (300 mL) est ajouté et le milieu réactionnel est agité pendant 30-40 minutes à température ambiante. Le précipité obtenu est filtré et lavé sur le filtre par de l'éther diéthylique (3 fois 40 mL) et enfin séché pendant 10 - 15 heures à température ambiante.

Un solide jaune (6,95 g, rendement 70 %) de point de fusion 122 °C est obtenu.

La pureté molaire est supérieure à 95 % (RMN ¹H).

Une Caractérisation RMN ¹H, ¹³C est présentée dans le tableau 1 suivant.

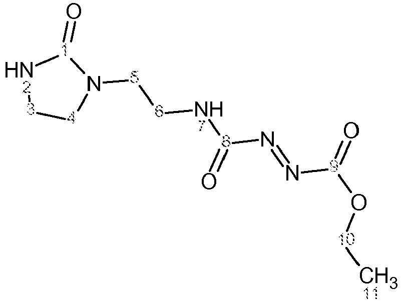

**Tableau 1**

| **Atome** | **δ ¹H (ppm) + mult.** | **δ ¹³C (ppm)** |
|---|---|---|
| **1** | - | 162.20 |
| **2** | 6.27/9.12* | - |
| **3** | 3.15 (t) | 37.46 |
| **4** | 3.32 (t) | 44.59 |
| **5** | 3.17 (t) | 42.41 |
| **6** | 3.31 (t) | 38.44 |
| **7** | 6.227/9.12* | - |
| **8** | - | 160.42/161.25 |
| **9** | - | 160.42/161.25 |
| **10** | 4.41 (q) | 65.24 |
| **11** | 1.28 (t) | 13.83 |

| | | |
|---|---|---|
| * Les protons 2 et 7 étant des groupements NH, leur déplacement chimique ¹H ne peut être exactement attribué. | | |

Solvant utilisé : DMSO - Calibration sur le signal du DMSO à 2.44 ppm en ¹H, 39.5 ppm en ¹³C

### Exemple 2 : préparation du N¹,N²-bis(2-(2-oxoimidazolidin-1-yl)ethyl)diazene-1,2-dicarboxamide en trois étapes à partir de l'UDETA.

### a) Préparation de N¹,N²-bis(2-(2-oxoimidazolidin-1-yl)ethyl)hydrazine-1,2-dicarboxamide (SI-BIM-02):

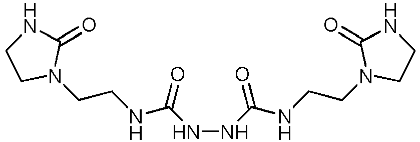

Au N-(2-(2-oxoimidazolidin-1-yl)ethyl)-1H-imidazole-1-carboxamide (4.46 g, 0,02 mol, pureté 86 % mol. par RMN) dans l'acétonitrile anhydre (100 mL)[produit de l'exemple 1] est ajouté en une fois l'hydrate d'hydrazine (0.50 g, 0.01 mol). Le milieu réactionnel est agité pendant 3 heures à 70-75 °C puis pendant 1-2 heures à température ambiante.
Le précipité est filtré et lavé par de l'acétonitrile (25 mL) et de l'éther de pétrole (50 mL, coupe 40/60°C) et enfin séché pendant 10 - 15 heures à température ambiante.
Un solide blanc (3,16 g, 0,009 mol., rendement 92 %) de point de fusion 232 °C est obtenu.
La pureté molaire est 90 % (RMN 1H).

Une Caractérisation RMN ¹H et ¹³C dans le DMSO est présentée dans le tableau 2 et dans D₂O, tableau 3 :

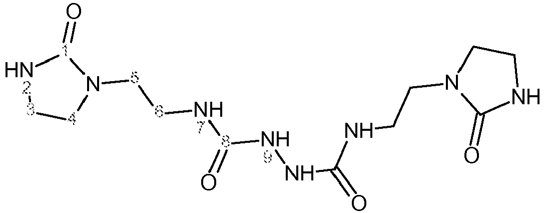

**Tableau 2**

| **Atome** | **δ ¹H (ppm) + mult.** | **δ ¹³C (ppm)** |
|---|---|---|
| **1** | - | 162.3 |
| **2** | ∼ 6.20 | - |
| **3** | 3.14 (t) | 37.5 |
| **4** | 3.29 (t) | 44.8 |
| **5** | 3.03 (t) | 43.2 |
| **6** | 3.03 (t) | 37.7 |
| **7** | ∼ 6.31/7.56 | - |
| **8** | - | 158.6 |
| **9** | ∼ 6.31/7.56 | - |

Solvant utilisé : DMSO - Calibration sur le signal du DMSO à 2.44 ppm en ¹H, 39.5 ppm en ¹³C

**Tableau 3**

| **Atome** | **δ ¹H (ppm) + mult.** | **δ ¹³C (ppm)** |
|---|---|---|
| **1** | - | 164.8 |
| **2** | - | - |
| **3** | 3.32 (t) | 38.0 |
| **4** | 3.47 (t) | 45.1 |
| **5** | 3.18 (t) | 42.9 |
| **6** | 3.25 (t) | 37.3 |
| **7** | - | - |
| **8** | - | 160.5 |
| **9** | - | - |

Solvant utilisé : D₂O - Calibration sur le signal de l'eau à 4,7 ppm en ¹H, sr=0 en ¹³C.

### b) Préparation de N¹,N²-bis(2-(2-oxoimidazolidin-1-yl)ethyl)diazene-1,2-dicarboxamide, composé selon l'invention

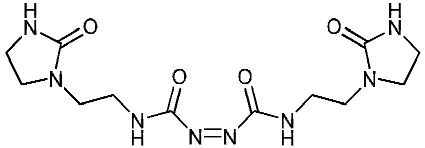

A un mélange de pyridine (0,237 g, 0,003 mol), de N¹,N²-bis(2-(2-oxoimidazolidin-1-yl)ethyl)hydrazine-1,2-dicarboxamide (1,03 g, 0,003 mol) et de dichlorométhane (50 mL) est ajouté en une fois la N-bromosuccinimide (0,534 g, 0,003 mol) à température ambiante. Le milieu réactionnel est agité pendant 1 heure à température ambiante. Le précipité est filtré et lavé sur le filtre par du dichlorométhane (10 mL) et séché pendant 1 heure. Le précipité est traité par l'eau (20 mL) pendant 15 minutes, filtré et lavé à nouveau avec de l'eau (20 mL) et de l'éther de pétrole (20 mL) et enfin séché pendant 10-15 heures à température ambiante.

Un solide jaune clair (0,62 g, 0,002 mol., rendement 61 %) de point de fusion 208 °C (décomposition) est obtenu. La pureté molaire est 90 % (RMN ¹H).

Une Caractérisation RMN ¹H et ¹³C est présentée dans le tableau 4

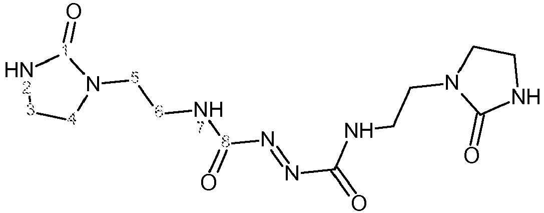

**Tableau 4**

| **Atome** | **δ ¹H (ppm) + mult.** | **δ ¹³C (ppm)** |
|---|---|---|
| **1** | - | 162.3 |
| **2** | ∼ 6.28/8.88 | - |
| **3** | 3.15 (t) | 37.5 |
| **4** | 3.32 (t) | 44.7 |
| **5** | 3.15 (t) | 42.6 |
| **6** | 3.28 (t) | 38.4 |
| **7** | ∼ 6.28/8.88 | - |
| **8** | - | 161.7 |

Solvant utilisé : DMSO - Calibration sur le signal du DMSO à 2.44 ppm en ¹H, 39.5 ppm en ¹³C

### Exemple 3 : préparation du N¹,N²-bis(2-(2-oxoimidazolidin-1-yl)ethyl)diazene-1,2-dicarboxamide en une étape à partir de l'UDETA

A un mélange de diisopropyl azo-1,2-dicarboxylate (1,01 g, 0,005 mol) dans l'éthanol (20 mL) est ajouté en une fois le 1-(2-aminoethyl)imidazolidin-2-one (1,29 g, 0,010 mol) à température ambiante. Le milieu réactionnel est agité pendant une heure à température ambiante. Le précipité est filtré et lavé par de l'éthanol (20 mL), de l'eau (50 mL) et de l'éther de pétrole (20 mL) puis séché pendant 10-15 heures à température ambiante.

Un solide jaune clair (1,26 g, 0,004 mol., rendement 74 %) de point de fusion 193 °C (dec) est obtenu.

La pureté par RMN ¹H est de 87% molaire.

## Revendications

1. Composé comprenant au moins un groupement Q, et au moins un groupement A reliés entre eux par au moins un groupement « espaceur » Sp dans lequel :
- Q comprend un groupe fonctionnel azo-dicarbonyle, répondant à la formule :
W-CO-N=N-CO-
dans laquelle W représente
un groupement de formule :
R'-Z-,
dans lequel :
• -Z représente un atome d'oxygène, de soufre ou un groupe -NH ou -NR',
• -R' représente un groupe alkyle en C₁-C₂₀,
ou
un groupement de formule :
-Sp'-A'
dans laquelle :
• Sp', identique ou différent de Sp est un groupement espaceur divalent reliant le groupe fonctionel azodicarbonylé à un autre groupe associatif A', et Sp' est une chaîne alkyle linéaire ou ramifiée en C₁-C₂₄, éventuellement comprenant un ou plusieurs hétéroatomes choisis parmi les atomes d'azote de soufre, de silicium.
• A', identique ou différent de A, est un groupe associatif comprenant au moins un atome d'azote, répondant à la formule (II) ci-après,
- A comprend un groupe associatif comprenant au moins un atome d'azote, répondant à la formule (II) suivante : 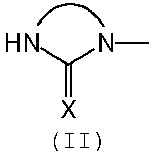 où X désigne un atome d'oxygène ou de soufre,
- le groupement espaceur Sp est une chaîne alkyle linéaire ou ramifiée en C₁-C₂₄, éventuellement comprenant un ou plusieurs hétéroatomes choisis parmi les atomes d'azote de soufre, de silicium ou d'oxygène.

2. Composé selon la revendication 1 **caractérisé en ce que** dans la formule (II) X désigne un atome d'oxygène.

3. Composé selon la revendication 1 ou 2 **caractérisé en ce que** le groupement espaceur Sp est une chaîne alkyle linéaire ou ramifiée en C₁-C₁₀, éventuellement comprenant un ou plusieurs hétéroatomes choisis parmi les atomes d'azote de soufre, de silicium ou d'oxygène.

4. Composé selon l'une quelconque des revendications 1 à 3 **caractérisé en ce que** R' représente un groupe alkyle en C₁-C₆.

5. Composé selon l'une quelconque des revendications précédentes, **caractérisé en ce que** le groupement espaceur Sp ou Sp' est une chaîne alkyle linéaire en C₁-C₆, éventuellement comprenant un ou plusieurs hétéroatomes choisis parmi les atomes d'azote de soufre, de silicium ou d'oxygène.

6. Composé selon l'une quelconque des revendications 2 à 5, **caractérisé en ce que** R' représente un groupe alkyle en C₁-C₄.

7. Composé selon l'une quelconque des revendications 2 à 6, **caractérisé en ce que** R' représente le méthyle ou l'éthyle.

8. Composé selon l'une quelconque des revendications précédentes, **caractérisé en ce qu'**il est représenté par la formule (VIII) ou (IX) :
R'-Z-CO-N=N-CO-Sp-A (VIII)
ou
A'-Sp'-CO-N=N-CO-Sp-A (IX)
dans lesquelles :
R', Z, Sp, A, Sp' et A' sont tels que définis dans les revendications précédentes.

9. Composé selon l'une quelconque des revendications précédentes, **caractérisé en ce qu'**il est représenté par la formule (X) ou (XI) suivante : 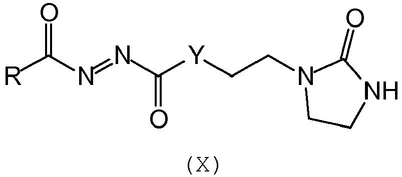 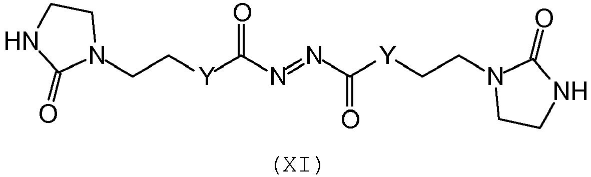 dans lesquelles :
- Y représente un groupe divalent choisi parmi un groupe méthylène, un atome d'oxygène, de soufre et un groupe -NH-, et
- R représente un groupe alcoxyle en C₁-C₆, de préférence un alcoxyle en C₁-C₄.

10. Composé selon la revendication 9 **caractérisé en ce que** R représente le méthoxyle ou l'éthoxyle.

11. Composé selon l'une quelconque des revendications précédentes **caractérisé en ce que** le groupement « espaceur » Sp ou Sp' est choisi parmi -(CH₂)y-, -NH-(CH₂)y-, -O-(CH₂) y-, y étant un entier de 1 à 6.

12. Composé selon l'une quelconque des revendications précédentes **caractérisé en ce qu'**il est choisi parmi les composés de formule (XII) à (XV) suivantes:

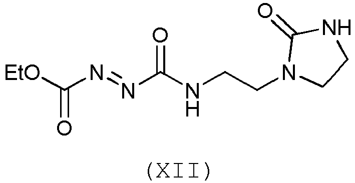

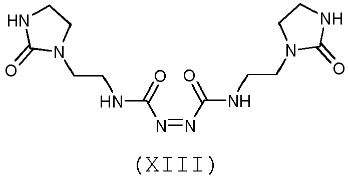

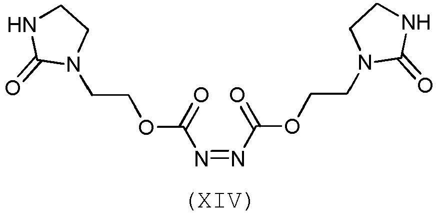

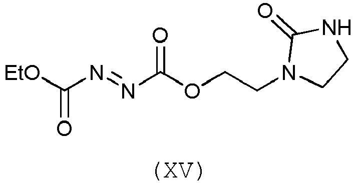

## Patentansprüche

1. Verbindung, umfassend mindestens eine Gruppierung Q und mindestens eine Gruppierung A, miteinander verbunden durch mindestens eine "Spacer"-Gruppierung Sp, in der:
- Q eine Azodicarbonyl-Funktion der Formel
W-CO-N=N-CO-
umfasst,
in der W
eine Gruppierung der Formel
R'-Z-
bedeutet,
in der:
• -Z ein Sauerstoffatom, ein Schwefelatom oder eine Gruppe -NH oder -NR' bedeutet,
• -R' eine C₁-C₂₀-Alkylgruppe bedeutet,
oder
eine Gruppierung der Formel:
-Sp'-A',
in der:
• Sp', die mit Sp identisch oder davon verschieden ist, eine divalente Spacer-Gruppierung ist, die die Azodicarbonyl-funktion mit einer anderen assoziativen Gruppe A' verbindet, und Sp' eine geradkettige oder verzweigte C₁-C₂₄-Alkylkette ist, die gegebenenfalls ein oder mehrere Heteroatome, ausgewählt aus Stickstoff-, Schwefel- und Siliziumatomen, umfasst,
• A', die mit A identisch oder davon verschieden ist, eine assoziative Gruppe ist, die mindestens ein Stickstoffatom umfasst, der Formel (II) unten,
• A eine assoziative Gruppe umfasst, die mindestens ein Stickstoffatom umfasst, der Formel (II) unten: 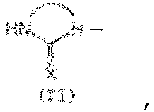 wobei X ein Sauerstoffatom oder ein Schwefelatom bedeutet,
• die Spacer-Gruppierung Sp eine geradkettige oder verzweigte C₁-C₂₄-Alkylkette ist, die gegebenenfalls ein oder mehrere Heteroatome, ausgewählt aus Stickstoff-, Schwefel-, Silizium- oder Sauerstoffatomen, umfasst.

2. Verbindung nach Anspruch 1, **dadurch gekennzeichnet, dass** X in Formel (II) ein Sauerstoffatom bedeutet.

3. Verbindung nach Anspruch 1 oder 2, **dadurch gekennzeichnet, dass** die Spacer-Gruppierung Sp eine geradkettige oder verzweigte C₁-C₁₀-Alkylkette, die gegebenenfalls ein oder mehrere Heteroatome, ausgewählt aus Stickstoff-, Schwefel-, Silizium- oder Sauerstoffatomen, umfasst, ist.

4. Verbindung nach einem der Ansprüche 1 bis 3, **dadurch gekennzeichnet, dass** R' eine C₁-C₆-Alkylgruppe bedeutet.

5. Verbindung nach einem der vorhergehenden Ansprüche, **dadurch gekennzeichnet, dass** die Spacer-Gruppierung Sp oder Sp' eine geradkettige C₁-C₆-Alkylkette, die gegebenenfalls ein oder mehrere Heteroatome, ausgewählt aus Stickstoff-, Schwefel-, Silizium- oder Sauerstoffatomen, umfasst, ist.

6. Verbindung nach einem der Ansprüche 2 bis 5, **dadurch gekennzeichnet, dass** R' eine C₁-C₄-Alkylgruppe bedeutet.

7. Verbindung nach einem der Ansprüche 2 bis 6, **dadurch gekennzeichnet, dass** R' Methyl oder Ethyl bedeutet.

8. Verbindung nach einem der vorhergehenden Ansprüche, **dadurch gekennzeichnet, dass** sie der Formel (VIII) oder (IX) entspricht:
R'-Z-CO-N=N-CO-Sp-A (VIII)
oder
A'-Sp'-CO-N-N-CO-Sp-A (IX)
, in denen:
R', Z, Sp, A, Sp' und A' wie in den vorhergehenden Ansprüchen definiert sind.

9. Verbindung nach einem der vorhergehenden Ansprüche, **dadurch gekennzeichnet, dass** sie der Formel (X) oder (XI) entspricht: 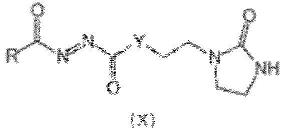 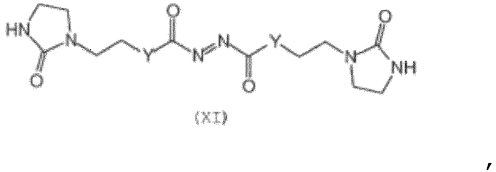 in denen:
- Y eine divalente Gruppe, ausgewählt aus einer Methylengruppe, einem Sauerstoffatom, einem Schwefelatom und einer Gruppe -NH-, bedeutet, und
- R eine C₁-C₆-Alkoxylgruppe, vorzugsweise eine C₁-C₄-Alkoxylgruppe, bedeutet.

10. Verbindung nach Anspruch 9, **dadurch gekennzeichnet, dass** R Methoxyl oder Ethoxyl bedeutet.

11. Verbindung nach einem der vorhergehenden Ansprüche, **dadurch gekennzeichnet, dass** die "Spacer"-Gruppierung Sp oder Sp' aus -(CH₂)y-, -NH-(CH₂)y- oder -O-(CH₂)y-ausgewählt ist, wobei y eine ganze Zahl von 1 bis 6 ist.

12. Verbindung nach einem der vorhergehenden Ansprüche, **dadurch gekennzeichnet, dass** sie aus den folgenden Verbindungen der Formeln (XII) bis (XV) ausgewählt ist:

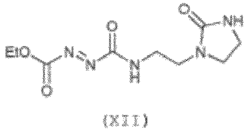

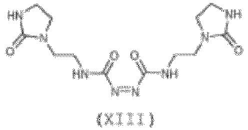

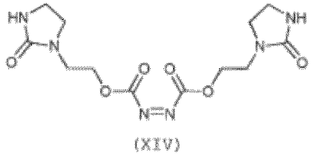

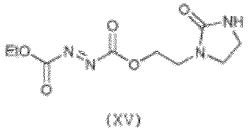

## Claims

1. Compound comprising at least one group Q, and at least one group A linked together by at least one "spacer" group Sp, in which:
- Q comprises an azodicarbonyl function, corresponding to the formula:
W-CO-N=N-CO-
in which W represents
a group of formula:
R'-Z-,
in which:
• -Z represents an oxygen or sulfur atom or an -NH or -NR' group,
• -R' represents a C₁-C₂₀ alkyl group,
or
a group of formula:
-Sp' -A'
in which:
• Sp', which may be identical to or different than Sp, is a divalent spacer group linking the azodicarbonyl functional group to another associative group A', and Sp' is a linear or branched C₁-C₂₄ alkyl chain, optionally comprising one or more hetereatoms chosen from nitrogen, sulfur or silicon atoms,
• A', which may be identical to or different than A, is an associative group comprising at least one nitrogen atom, corresponding to formula (II) below,
- A comprises an associative group comprising at least one nitrogen atom, corresponding to formula (II) below: 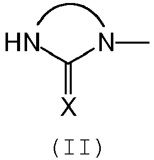 wherein X denotes an oxygen or sulfur atom,
- the spacer group Sp is a linear or branched C₁-C₂₄ alkyl chain, optionally comprising one or more heteroatoms chosen from nitrogen, sulfur, silicon or oxygen atoms.

2. Compound according to Claim 1, **characterized in that** in formula (II) X denotes an oxygen atom.

3. Compound according to Claim 1 or 2, **characterized in that** the spacer group Sp is a linear or branched C₁-C₁₀ alkyl chain, optionally comprising one or more heteroatoms chosen from nitrogen, sulfur, silicon or oxygen atoms.

4. Compound according to any one of Claims 1 to 3, **characterized in that** R' represents a C₁-C₆ alkyl group.

5. Compound according to any one of the preceding claims, **characterized in that** the spacer group Sp or Sp' is a linear C₁-C₆ alkyl chain, optionally comprising one or more heteroatoms chosen from nitrogen, sulfur, silicon or oxygen atoms.

6. Compound according to any one of Claims 2 to 5, **characterized in that** R' represents a C₁-C₄ alkyl group.

7. Compound according to any one of Claims 2 to 6, **characterized in that** R' represents methyl or ethyl.

8. Compound according to any one of the preceding claims, **characterized in that** it is represented by formula (VIII) or (IX) :
R'-Z-CO-N=N-CO-Sp-A (VIII)
or
A'-Sp'-CO-N=N-CO-Sp-A (IX)
in which:
R', Z, Sp, A, Sp' and A' are as defined in the preceding claims.

9. Compound according to any one of the preceding claims, **characterized in that** it is represented by formula (X) or (XI) below: 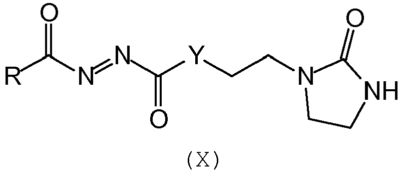 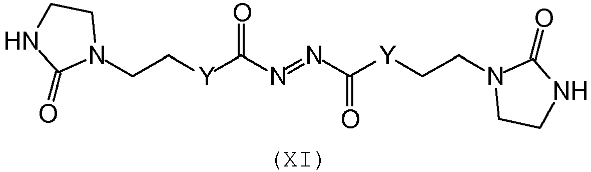 in which:
- Y represents a divalent group chosen from a methylene group, an oxygen atom, a sulfur atom and an -NH- group, and
- R represents a C₁-C₆ alkoxyl group, preferably a C₁-C₄ alkoxyl group.

10. Compound according to Claim 9, **characterized in that** R represents methoxyl or ethoxyl.

11. Compound according to any one of the preceding claims, **characterized in that** the "spacer" group Sp or Sp' is chosen from -(CH₂)_{y}-, -NH-(CH₂)_{y}- and -O-(CH₂)_{y}-, y being an integer from 1 to 6.

12. Compound according to any one of the preceding claims, **characterized in that** it is chosen from the compounds of formulae (XII) to (XV) below:
